(19) Europäisches Patentamt
European Patent Office
Office européen des brevets

(11) Veröffentlichungsnummer : **0 062 905**
**B1**

(12) # EUROPÄISCHE PATENTSCHRIFT

(45) Veröffentlichungstag der Patentschrift : **13.03.85**

(21) Anmeldenummer : **82103032.7**

(22) Anmeldetag : **08.04.82**

(51) Int. Cl.⁴ : **C 07 D263/58**, C 07 D277/68, C 07 D413/12, C 07 D417/12, A 01 N 43/74

(54) **Heterocyclische Phenylether, Verfahren zu deren Herstellung und diese enthaltende herbizide Mittel.**

(30) Priorität : **15.04.81 DE 3115152**

(43) Veröffentlichungstag der Anmeldung : **20.10.82 Patentblatt 82/42**

(45) Bekanntmachung des Hinweises auf die Patenterteilung : **13.03.85 Patentblatt 85/11**

(84) Benannte Vertragsstaaten : **AT BE CH DE FR GB IT LI NL**

(56) Entgegenhaltungen :
**GB-A- 2 046 753**
**US-A- 4 130 413**
**Die Akte enthält technische Angaben, die nach dem Eingang der Anmeldung eingereicht wurden und die nicht in dieser Patentschrift enthalten sind.**

(73) Patentinhaber : **HOECHST AKTIENGESELLSCHAFT**
**Postfach 80 03 20**
**D-6230 Frankfurt am Main 80 (DE)**

(72) Erfinder : **Handte, Reinhard, Dr.**
**Breckenheimer Strasse 45**
**D-6238 Hofheim am Taunus (DE)**
Erfinder : **Bauer, Klaus, Dr.**
**Kolpingstrasse 7**
**D-6054 Rodgau (DE)**
Erfinder : **Bieringer, Hermann, Dr.**
**Eichenstrasse 26**
**D-6239 Eppstein/Taunus (DE)**

**EP 0 062 905 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Aus US-A-4 130 413 und GB-A-2 046 753 sind bereits zahlreiche Derivate von Benzoxazolyloxy- und Benzthiazolyloxyphenoxy-propionsäuren mit ausgeprägter herbizider Wirksamkeit besonders gegen Ungräser bekannt.

Gegenstand der Erfindung sind neue heterocyclisch substituierte 4-Oxyphenoxyalkancarbonsäurederivate der allgemeinen Formel I

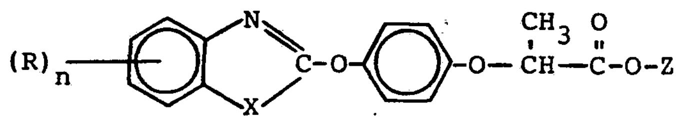

(I)

worin

R Halogen,

n 0, 1 oder 2,

X 0 oder S

und

Z eine Gruppe der Formeln

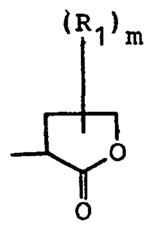

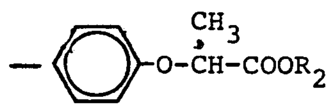

oder Morpholinomethyl oder -ethyl,

$R_1$ ($C_1$-$C_4$) Alkyl,

m 0-2,

$R_2$ H, ($C_1$-$C_4$)-Alkyl oder ein Kationäquivalent

bedeuten.

Bevorzugte Verbindungen der allgemeinen Formel I sind solche, in denen R Halogen (insbesondere F, Cl, Br) und n null oder 1 sind und Z die angegebene Bedeutung hat.

Kationenäquivalente in $R_2$-Stellung sind bevorzugt $Na^{\oplus}$ und $K^{\oplus}$.

Die Verbindungen der allgemeinen Formel I besitzen ein Asymmetriezentrum und werden bei ihrer Herstellung gewöhnlich als Racemate erhalten. Die Erfindung umfaßt jedoch auch die isolierten optischen Antipoden und dabei insbesondere deren D-Formen.

Gegenstand der Erfindung ist ferner ein Verfahren zur Herstellung der Verbindungen der allgemeinen Formel I, das dadurch gekennzeichnet ist, daß man

a) Verbindungen der allgemeinen Formel II

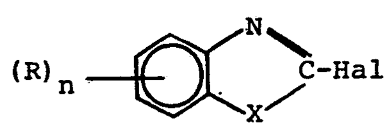

(II)

worin Hal ein Halogenatom darstellt, mit Verbindungen der allgemeinen Formel III

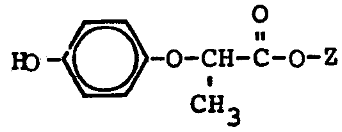

(III)

oder

b) Verbindungen der allgemeinen Formel IV

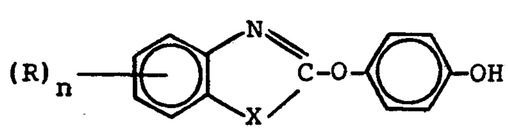

(IV)

mit Verbindungen der allgemeinen Formel (V)

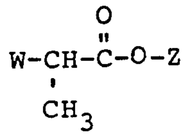

(V)

worin W für Halogen (vorzugsweise Chlor oder Brom) oder den Tosylrest steht, oder

c) Verbindungen der allgemeinen Formel VI

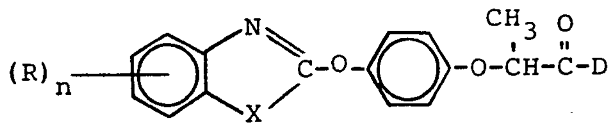

(VI)

mit Verbindungen der Formel B-Z (VII), wobei jeweils einer der Reste D und B Halogen und der andere die Gruppe —OH derstellt, umsetzt.

Die Umsetzungen nach a) bis c) erfolgen in der für den Fachmann geläufigen Weise, wobei entweder in Gegenwart von säurebindenden Mitteln gearbeitet wird oder die Ausgangsstoffe III, IV bzw. VI (VII) in Form ihrer Salze eingesetzt werden, die ggf. in situ erzeugt werden können. Die allgemeinen Verfahren sind z. B. in der US-PS 4 130 413 näher beschrieben.

Die Ausgangsverbindungen der Formeln allgemeinen II-VII sind bekannt bzw. lassen sich nach bekannten Verfahren herstellen. Bei Verwendung von optisch aktiven Ausgangsmaterialien der allgemeinen Formeln III, V oder VI ist es möglich, optische Isomere der erfindungsgemäßen Verbindungen (bevorzugt die D-Form) der allgemeinen Formel I in hohen optischen Reinheiten herzustellen.

Die erfindungsgemäßen Verbindungen der allgemeinen Formel I sind im Vor- und Nachauflaufverfahren gegen ein breites Spektrum von ein- und mehrjährigen Schadgräsern sehr gut wirksam, gleichzeitig werden sie jedoch von zweikeimblättrigen Kulturpflanzen sowie einigen Getreidearten vorzüglich toleriert. Die Verbindungen sind daher zur selektiven Bekämpfung von ein- und mehrjährigen Schadgräsern in Kulturpflanzen geeignet. Solche Schadgräser sind beispielsweise Wildhafer (Avena), Fuchsschwanz (Alopecurus spp.), Rispengras (Poa spp.), Raygras (Lolium spp.), ein- und mehrjährige Wildhirsen (Echinochloa spp., Setaria spp., Digitaria spp., Panicum spp., Sorghum spp.), Bermudagras (Cynodon spp.) und Quecke (Agropyron spp.).

Gegenstand der Erfindung sind daher auch herbizide Mittel, die dadurch gekennzeichnet sind, daß sie eine herbizid wirksame Menge einer Verbindung der allgemeinen Formel I neben üblichen Zusatz- und Formulierungshilsmitteln enthalten.

Die erfindungsgemäßen Mittel enthalten die Wirkstoffe der allgemeinen Formel I im allgemeinen zu 2-95 Gew.-%. Sie können als benetzbare Pulver, emulgierbare Konzentrate, versprühbare Lösungen, Stäubemittel oder Granulate in den üblichen zubereitungen angewendet werden.

Benetzbare Pulver sind in Wasser gleichmäßig dispergierbare Präparate, die neben dem Wirkstoff außer einem Verdünnungs- oder Inertstoff noch Netzmittel, z. B. polyoxethylierte Alkylphenole, polyoxyethylierte Oleyl-, Stearylamine, Alkyl- oder Alkylphenyl-sulfonate und Dispergiermittel, z. B. ligninsulfonsaures Natrium, 2,2'-dinaphthylmethan-6,6'-disulfonsaures Natrium, oder auch oleylmethyltaurinsaures Natrium enthalten.

Emulgierbare Konzentrate werden durch Auflösen des Wirkstoffes in einem organischen Lösungsmittel, z. B. Butanol, Cyclohexanon, Dimethylformamid, Xylol oder auch höhersiedenden Aromaten und Zusatz eines nichtionischen Netzmittels, beispielsweise eines polyoxethylierten Alkylphenols oder eines polyoxethylierten Oleyl- oder Stearylamins, erhalten.

Stäubemittel erhält man durch Vermahlen des Wirkstoffes mit fein verteilten, festen Stoffen, z. B. Talkum, natürlichen Tonen, wie Kaolin, Bentonit, Pyrophillit oder Diatomeenerde.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z. B. Polyvinylalkohol, polyacrylsaurem Natrium, oder auch Mineralölen auf die Oberfläche von Trägerstoffen, wie Sand, Kaolinite oder von granuliertem Inertmaterial. Auch können geeignete Wirkstoffe in der für die Herstellung von Düngemittelgranalien üblichen Weise — gewünschtenfalls in Mischung mit Düngemitteln — hergestellt werden.

Bei den herbiziden Mitteln können die Konzentrationen der Wirkstoffe in den handelsüblichen Formulierungen verschieden sein. In benetzbaren Pulvern variiert die Wirkstoffkonzentration z. B. zwischen etwa 10 % und 95 %, der Rest besteht aus den oben angegebenen Formulierungszusätzen. Bei emulgierbaren Konzentraten ist die Wirkstoffkonzentration etwa 10 % bis 80 %. Staubförmige Formulierungen enthalten meistens 5 % bis 20 % an Wirkstoff. Bei Granulaten hängt der Wirkstoffgehalt z. T. davon

ab, ob die wirksame Verbindung flüssig oder fest vorliegt und welche Granulierhilfsmittel, Füllstoffe usw. verwendet werden.

Zur Anwendung werden die handelsüblichen Konzentrate gegebenenfalls in üblicher Weise verdünnt, z. B. bei benetzbaren Pulvern und emulgierbaren Konzentraten mittels Wasser. Staubförmige und granulierte Zubereitungen sowie versprühbare Lösungen werden vor der Anwendung nicht mehr mit weiteren inerten Stoffen verdünnt. Mit den äußeren Bedingungen wie Temperatur, Feuchtigkeit u. a. variiert die erforderliche Aufwandmenge. Sie kann innerhalb weiter Grenzen schwanken, z. B. zwischen 0,05 und 10,0 kg/ha oder mehr Aktivsubstanz, vorzugsweise liegt sie jedoch zwischen 0,1 und 5 kg/ha.

Die erfindungsgemäßen Wirkstoffe können mit anderen Herbiziden, Insektiziden und Fungiziden kombiniert werden.

Bei der Kombination mit Herbiziden eignen sich insbesondere die gegen Dikotyledonen wirksamen Präparate aus der Gruppe der Nitrodiphenylether sowie Phenmedipham, Desmedipham, Bentazon, Metamitron und folgender Harnstoff

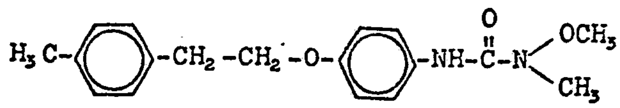

Formulierungsbeispiele

Beispiel A

Ein emulgierbares Konzentrat wird erhalten aus

15 Gew.-Teilen Wirkstoff
75 Gew.-Teilen Cyclohexanon als Lösungsmittel
und
10 Gew.-Teilen oxethyliertes Nonylphenol (10 AeO) als Emulgator.

Beispiel B

Ein in Wasser leicht dispergierbares benetzbares Pulver wird erhalten, indem man

25 Gew.-Teile Wirkstoff
64 Gew.-Teile kaolinhaltiges Quarz als Inertstoff
10 Gew.-Teile ligninsulfonsaures Kalium
und
1 Gew.-Teil oleylmethyltaurinsaures Natrium als Netz- und Dispergiermittel
mischt und in einer Stiftmühle mahlt.

Beispiel C

Ein Stäubemittel wird erhalten, indem man

10 Gew.-Teile Wirkstoff
und
90 Gew.-Teile Talkum als Inertstoff
mischt und in einer Schlagmühle zerkleinert.

Beispiel D

Ein Granulat besteht z. B. aus
2-15 Gew.-Teilen Wirkstoff
98-85 Gew.-Teilen inerten Granulatmaterialien, wie z. B. Attapulgit, Bimsstein und Quarzsand.

Herstellungsbeispiele

Beispiel 1

2-Oxotetrahydrofuranyl-(3)-2-[4-(6-chlor-2-benzthiazolyloxy)-phenoxy]-propanoat

21 g (0,06 Mol) 2-[4-(6-Chlor-2-benzthiazolyloxy-phenoxy]-propionsäure werden mit 10,4 g (0,075 Mol) Kaliumcarbonat in 100 ml Aceton vorgelegt und ca. 1/2 Stunde bei 45 °C nachgerührt, es resultiert

eine dicke Paste. Es werden anschließend innerhalb von 1/2 Stunde 11,6 g (0,07 Mol) 2-Brombutyrolacton in 50 ml Aceton zugetropft. Nach der Zugabe wird das Reaktionsgemisch dünnflüssig, der Umsatz wird dünnschichtchromatographisch verfolgt, nach ca. 5 Stunden beträgt er ca. 70 %. Insgesamt wird die Mischung 16 Stunden bei ca. 50 °C gerührt. Nach Abkühlen wird vom Salz abgesaugt, Aceton abdestilliert, der Rückstand in ca. 200 ml Toluol aufgenommen und mit Wasser und gesättigter Natriumbicarbonatlösung gewaschen. Nach Trocknen wird Toluol abdestilliert und der verbleibende Rückstand eine Stunde bei ca. 70 °C im Vakuum (0,05 mbar) getrocknet. Es resultieren nach dem Trocknen 22 g (91,3 % der Theorie) eines sehr zähen Oels, welches durch $^1$H Kernresonanzspektroskopie als 2-Oxotetrahydrofuranyl-(3)-2-[4-(6-benzthiazolyloxy)-phenoxy]-propanoat identifiziert wird.

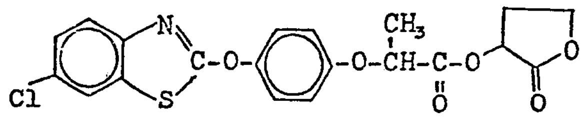

Beispiel 2

(D+)-2-Oxotetrahydrofuranyl-(3)-2-[4-(6-chlor-2-benzthiazolyloxy)-phenoxy]-propanoat

Wird analog Beispiel 1 verfahren und anstelle des Racemats die D-Form der Säure angesetzt, so kommt man mit gleicher Ausbeute zu (D+)-2-Oxotetrahydrofuranyl-(3)-2-[4-(6-chlor-2-benzthiazolyloxy)-phenoxy]-propanoat. Die Identifiziertung erfolgt durch $^1$H-Kernresonanzspektroskopie und durch Messung des Drehwinkels in Chloroform.

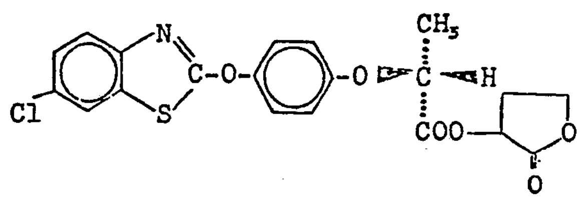

Beispiel 3

2-Morpholinoethyl-2-[4-(6-chlor-2-benzthiazolyloxy)-phenoxy]-propanoat

35 g (0,1 Mol) 2-[4-(6-Chlor-2-benzthiazolyloxy)-phenoxy]-propionsäure werden in 250 ml trockenem Toluol suspendiert und bei 60 °C innerhalb von 1 Stunde mit 14,3 g (0,12 Mol) Thionylchlorid verdünnt mit 50 ml Toluol, versetzt. Nach der Zugabe wird insgesamt 7 Stunden bei 70 °C nachgerührt, anschließend wird überschüssiges Thionylchlorid mit ca. 100 ml Toluol abdestilliert, es resultiert eine klare Lösung des Säurechlorids. Der Ansatz wird abgekühlt, mit 100 ml trockenem Toluol versetzt, anschließend werden 12 g (0,1 Mol) Morpholinethanol gelöst in 100 ml Toluol zugegeben, nach der Zugabe wird 1 h bei 50 °C nachgerührt, es resultiert zunächst das Hydrochlorid der Verbindung, durch Zugabe von 200 ml gesättigter Bicarbonatlösung und anschließende Wasserwäsche erhält man eine toluolische Produktlösung, die abgetrennt wird. Nach Abdestillieren von Toluol erhält man einen zähen Rückstand, der eine Stunde bei 100 °C im Vakuum (0,05 mbar) getrocknet wird. Nach dem Trocknen erhält man 40,4 g (89,8 % der Theorie) an 2-Morpholinoethyl-2-4-(6-chlor-2-benzthiazolyloxy)-phenoxy-propanoat als zähes Oel. Die Struktur der Verbindung wird mit Hilfe der $^1$H-Kernresonanzspektroskopie nachgewiesen.

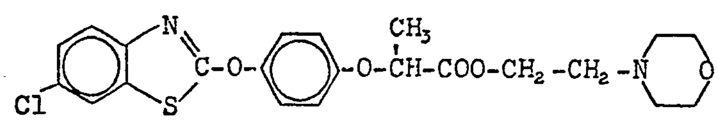

Beispiel 4

4-(Ethoxycarbonyl-1-ethoxy)-phenyl-2-[4-(6-chlor-2-benzthiazolyloxy)-phenoxy]-propanoat

17,5 g (0,05 mol 2-[4-(6-Chlor-2-benzthiazolyloxy)-phenoxy]-propionsäure werden in 100 ml trockenem Toluol gelöst und mit 7,15 g (0,06 mol) Thionylchlorid unter Erwärmen in das Säurechlorid überführt. Nach Abdestillieren von überschüssigem Thionylchlorid und ca. 50 ml Toluol werden 10,5 g (0,05 mol) Ethyl-2-(4-Hydroxyphenoxy)-propanoat und 6,1 g (0,06 mol) Triethylamin in 100 ml Toluol zugetropft. Nach der Zugabe wird 1 h bei 50 °C nachgerührt und abgekühlt. Das Reaktionsprodukt wird zweimal mit je 50 ml 5 %iger Natronlauge und zweimal mit je 100 ml Wasser gewaschen. Nach Trocknen und Einengen

verbleiben als öliger Rückstand 26 g (96 % d. Th.) 4-(Ethoxycarbonyl-1-ethoxy)-phenyl-2-[4-(6-chlor-2-benzthiazolyloxy)-phenoxy]-propanoat. Die Identifizierung erfolgte durch $^1$H-Kernresonanzspektroskopie.

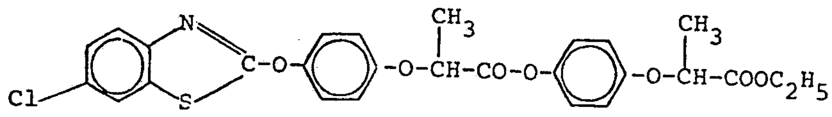

Analog den vorigen Beispielen lassen sich folgende Verbindungen herstellen.

| lfd. Nr. | $(R)_n$ | X | Z | Fp., Kp. $[n_D]$ |
|---|---|---|---|---|
| 5 | 6-Cl | O | 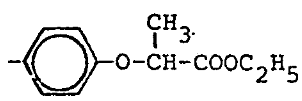 | $^1$H-NMR-Spektrum (Tab. 2) |
| 6 | 6-F | S | $-CH_2-CH_2-$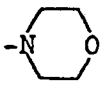 | |
| 7 | 6-Cl | S | $-CH_2-CH_2-$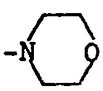 | |

Tabelle 2

$^1$H-NMR-Daten zur Charakterisierung einzelner Verbindungen aus der Reihe der Herstellungsbeispiele, Aufnahme bei 60 MHz, Lösungsmittel $CDCl_3$; Chemische Verschiebung δ (ppm) relativ zum Standard TMS (in Klammern: Multiplizität und relative Intensität der Signale)

| Bsp. Nr. | $-CH_3$ | $-CH_2-$ | $-\overset{\vert}{C}H-$ | Aromaten |
|---|---|---|---|---|
| 1, 2 | 1.65 (d) | 2.9-2 (m)<br>4.9-4.2 (m) | 4.8 (q)<br>4.4 (t) | 6.6 - 7.2 (m, 7) |
| 4 | 1.2 (t)<br>1.65 (d)<br>1.75 (d) | 4.2 (q) | 4.65 (q)<br>4.9 (q) | 6.6 - 7.8 (m, 11) |
| 5 | 1.2 (t)<br>1.6 (d)<br>1.75 (d) | 4.2 (q) | 4.7 (q)<br>4.9 (q) | 6.65 - 7.6 (m, 11) |

d = Duplett
t = Triplett
q = Quadruplett
m = Multiplett

## Biologische Beispiele

### Beispiel 1

Vorauflaufbehandlung

Samen von Gräsern wurden in Töpfen ausgesät und die als Spritzpulver bzw. als Emulsionskonzentrate formulierten erfindungsgemäßen Präparate in verschiedenen Dosierungen auf die Erdoberfläche gesprüht. Anschließend wurden die Töpfe für 4 Wochen in einem Gewächshaus aufgestellt und das Resultat der Behandlung (ebenso wie bei den folgenden Beispielen) durch eine Bonitierung nach folgendem Schema (siehe Tabelle) festgehalten.

Die erfindungsgemäßen Präparate zeigten eine gute Wirkung gegen einjährige und zum Teil auch mehrjährige Schadgräser :

| Bsp. | Dosis a.i./ha | AVF | ALM | SAL | LOM | ECG | AGR | CND |
|---|---|---|---|---|---|---|---|---|
| 1 | 2,4 kg | 5 | 5 | 5 | 5 | 5 | 5 | 5 |
| | 0,6 kg | 5 | 5 | 5 | 5 | 5 | - | - |
| 4 | 2,4 kg | 5 | 5 | 5 | 5 | 5 | 5 | - |
| | | 5 | 5 | 5 | 5 | 5 | - | - |
| 5 | 2,4 kg | 5 | 5 | 5 | 5 | 5 | - | - |
| | 0,6 kg | 4 | 4 | 5 | 4 | 5 | - | - |

AVF : Flughafer
ALM : Ackerfuchsschwanz
SAL : Borstenhirse
LOM : Raygras
ECG : Hühnerhirse
AGR : Quecke
CND : Bermudagras
a. i. = Aktivsubstanz

## Beispiel II

Nachauflaufbehandlung

Samen von Gräsern wurden in Töpfen ausgesät und im Gewächshaus angezogen. 3 Wochen nach der Aussaat wurden die als Spritzpulver bzw. als Emulsionskonzentrate formulierten erfindungsgemäßen Präparate in verschiedenen Dosierungen auf die Pflanzen gesprüht und nach 4 Wochen Standzeit im Gewächshaus die Wirkung der Präparate bonitiert.

Die erfindungsgemäßen Mittel waren gut gegen ein breites Spektrum von einjährigen Schadgräsern herbizid wirksam. Einige Präparate bekämpften ferner auch die mehrjährigen Schadgräser Cynodon dactylon, Sorghum halepense sowie Agropyron repens ; genannt sei die Verbindung von Beispiel 1.

| Dosis | AVF | ALM | SAL | LOM | ECG | AGR | CND |
|---|---|---|---|---|---|---|---|
| 2,4 kg a.i./kg | 5 | 5 | 5 | 5 | 5 | 5 | - |
| 0,6 kg a.i./kg | 5 | 5 | 5 | 5 | 5 | - | - |

## Beispiel III

Kulturpflanzenverträglichkeit

In weiteren Versuchen im Gewächshaus wurden Samen einer größeren Anzahl von Kulturpflanzen in Töpfen ausgelegt. Ein Teil der Töpfe wurde sofort behandelt, die übrigen wurden im Gewächshaus aufgestellt, bis die Pflanzen 2 bis 3 echte Blätter entwickelt hatten und dann mit erfindungsgemäßen substanzen besprüht.

Die Ergebnisse, die 4 bis 5 Wochen nach Applikation festgestellt wurden, zeigen, daß die erfindungsgemäßen Substanzen zweikeimblättrige Kulturen im Vor- und Nachauflauf-Verfahren selbst mit 2,5 kg/ha völlig oder fast völlig ungeschädigt lassen. Einige Substanzen schonen darüber hinaus auch Gramineen-Kulturen wie Gerste, Sorghum, Mais, Weizen oder Reis. Die Substanzen sind somit bezüglich der in den vorigen Beispielen beschriebenen Unkrautwirkung hoch selektiv.

Tabelle

Bonitierung in %-Werten und Zahlen

| Wertzahl | Schadwirkung in % bei Unkräutern und Kulturpflanzen |
|---|---|
| 1 | 0 - 20 % |
| 2 | 20 - 40 % |
| 3 | 40 - 60 % |
| 4 | 60 - 80 % |
| 5 | 80 - 100 % |

**Ansprüche** (für die Vertragsstaaten : BE, CH, DE, FR, GB, IT, LI, NL)

1. Verbindungen der allgemeinen Formel I

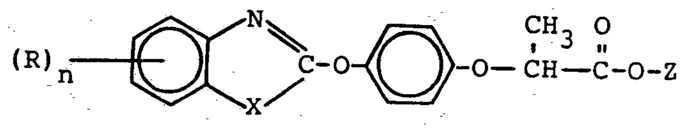

(I)

worin
R Halogen,
n 0, 1 oder 2,
X O oder S,
und
Z eine Gruppe der Formel

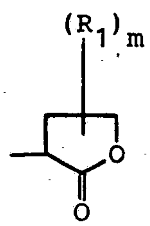

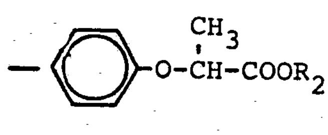

oder Morpholinomethyl oder -ethyl
$R_1$ $(C_1$-$C_4)$Alkyl,
m 0-2,
$R_2$ H, $(C_1$-$C_4)$-Alkyl oder ein Kationäquivalent,
bedeuten.

2. Verfahren zur Herstellung der Verbindungen der allgemeinen Formel I, dadurch gekennzeichnet, daß man

a) Verbindungen der allgemeinen Formel II

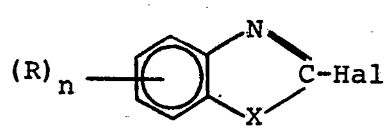

(II)

worin Hal ein Halogenatom darstellt, mit Verbindungen der allgemeinen Formel III

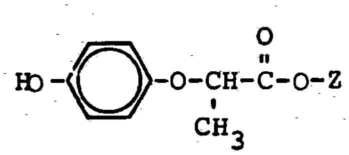

(III)

oder

b) Verbindungen der allgemeinen Formel IV

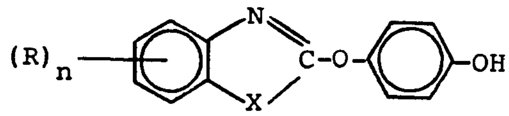 (IV)

mit Verbindungen der allgemeinen V

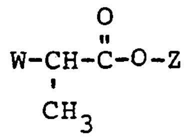 (V)

worin W für Halogen (vorzugsweise Chlor oder Brom) oder den Tosylrest steht, oder

c) Verbindungen der allgemeinen Formel VI

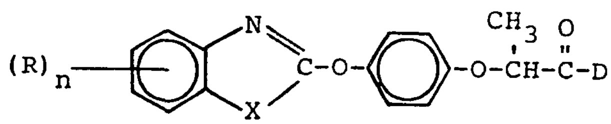 (VI)

mit Verbindungen der Formel B-Z (VII), wobei jeweils einer der Reste D und B Halogen und der andere die Gruppe —OH darstellt, umsetzt.

3. Herbizide Mittel, gekennzeichnet durch einen Gehalt an Verbindungen der allgemeinen Formel I.

4. Verwendung von Verbindungen der allgemeinen Formel I zur Bekämpfung von Schadgräsern.

5. Verfahren zur Bekämpfung von unerwünschten Gräsern in Kulturpflanzen, dadurch gekennzeichnet, daß man auf die unerwünschten Gräser bzw. ihren Lebensraum eine herbizid wirksame Menge einer Verbindung der allgemeinen Formel I aufbringt.

**Ansprüche** (für den Vertragsstaat AT)

1. Verfahren zur Herstellung von Verbindungen der allgemeinen Formel I

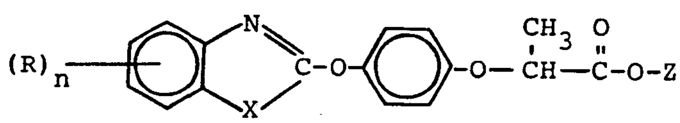 (I)

worin

R Halogen,

n 0, 1 oder 2,

X O oder S,

und

Z eine Gruppe der Formel

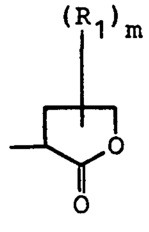

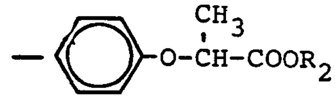

oder Morpholinomethyl oder -ethyl

$R_1$ $(C_1$-$C_4)$Alkyl,

m 0-2,

$R_2$ H, $(C_1$-$C_4)$-Alkyl oder ein Kationäquivalent bedeuten,

bedeuten, dadurch gekennzeichnet, daß man

a) Verbindungen der allgemeinen Formel II

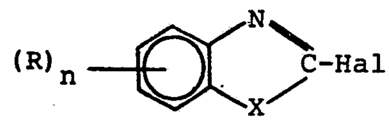 (II)

worin Hal ein Halogenatom darstellt, mit Verbindungen der allgemeinen Formel III

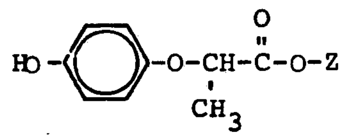 (III)

oder

b) Verbindungen der allgemeinen Formel IV

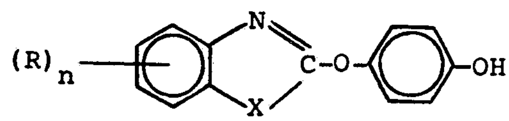 (IV)

mit Verbindungen der allgemeinen Formel V

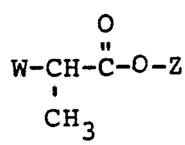 (V)

worin W für Halogen (vorzugsweise Chlor oder Brom) oder den Tosylrest steht, oder

c) Verbindungen der allgemeinen Formel VI

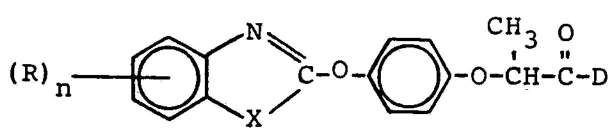 (VI)

mit Verbindungen der Formel B-Z (VII), wobei jeweils einer der Reste D und B Halogen und der andere die Gruppe —OH darstellt, umsetzt.

2. Herbizide Mittel, gekennzeichnet durch einen Gehalt an Verbindungen der allgemeinen Formel I.

3. Verwendung von Verbindungen der allgemeinen Formel I zur Bekämpfung von Schadgräsern.

4. Verfahren zur Bekämpfung von unerwünschten Gräsern in Kulturpflanzen, dadurch gekennzeichnet, daß man auf die unerwünschten Gräser bzw. ihren Lebensraum eine herbizid wirksame Menge einer Verbindung der allgemeinen Formel I aufbringt.

**Claims** (for the Contracting States ; BE, CH, DE, FR, GB, IT, LI, NL)

1. Compounds of the general formula I

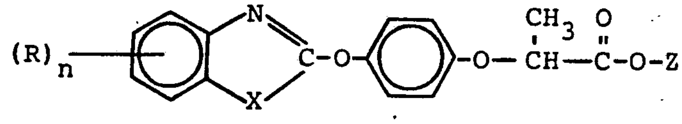 (I)

in which

R denotes halogen,

n denotes 0, 1 or 2,

X denotes O or S

and

Z denotes a group of the formula

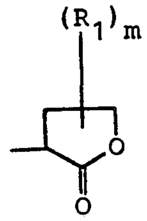 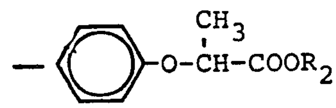

or morpholinomethyl or -ethyl,

$R_1$ denotes ($C_1$-$C_4$)-alkyl,

m denotes 0-2, and

$R_2$ denotes H, ($C_1$-$C_4$)-alkyl or a cation equivalent.

2. A process for the preparation of compounds of the general formula I, which comprises

a) reacting compounds of the general formula II

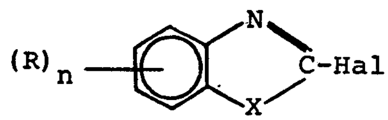 (II)

in which Hal represents a halogen atom, with compounds of the general formula III

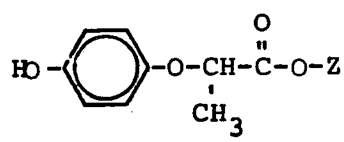 (III)

or

b) reacting compounds of the general formula IV

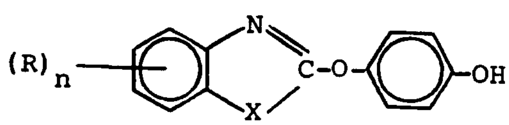 (IV)

with compounds of the general formula V

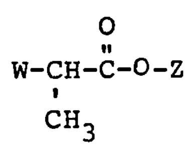 (V)

in which W represents halogen (preferably chlorine or bromine) or the tosyl radical, or

c) reacting compounds of the general formula VI

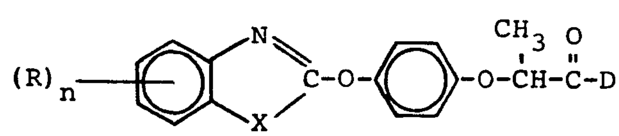 (VI)

with a compound of the formula B-Z (VII), wherein one of the radicals D and B represents halogen and the other group —OH.

3. Herbicidal agents which are characterized by a content of compounds of the general formula I.

4. The use of compounds of the general formula I for combating gramineous weeds.

5. A process for combating undesired grasses in crop plants, which comprises applying a herbicidally effective quantity of a compound of the general formula I to the undesired grasses or to their habitat.

**Claims** (for the Contracting State AT)

1. A process for the preparation of a compound of the general formula I

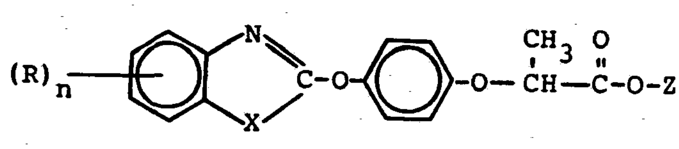 (I)

in which

R denotes halogen,

n denotes 0, 1 or 2,

X denotes O or S

and

Z denotes a group of the formula

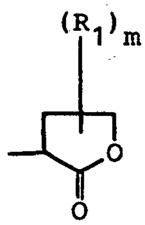

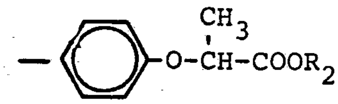

or morpholinomethyl or -ethyl,

$R_1$ denotes $(C_1$-$C_4)$-alkyl,

m denotes 0-2, and

$R_2$ denotes H, $(C_1$-$C_4)$-alkyl or a cation equivalent, which comprises

a) reacting compounds of the general formula II

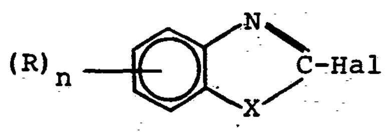 (II)

in which Hal represents a halogen atom, with compounds of the general formula III

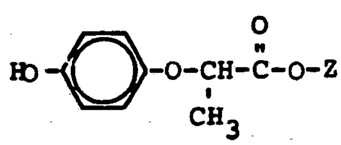 (III)

or

b) reacting compounds of the general formula IV

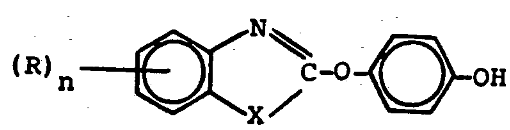 (IV)

with compounds of the general formula V

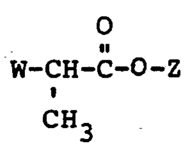 (V)

in which W represents halogen (preferably chlorine or bromine) or the tosyl radical, or

c) reacting a compound of the general formula VI

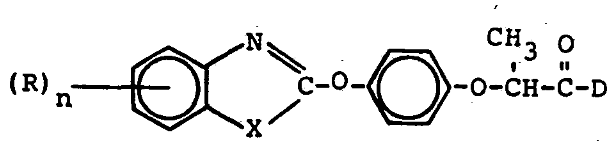 (VI)

with compounds of the formula B-Z (VII), wherein one of the radicals D and B represents halogen and the other group —OH.

2. Herbicidal agents which are characterized by a content of compounds of the general formula I.

3. The use of compounds of the general formula I for combating gramineous weeds.

4. A process for combating undesired grasses in crop plants, which comprises applying a herbicidally effective quantity of a compound of the general formula I to the undesired grasses or to their habitat.

**Revendications** (pour les Etats contractants : BE, CH, DE, FR, GB, IT, LI, NL)

1. Composés répondant à la formule générale (I)

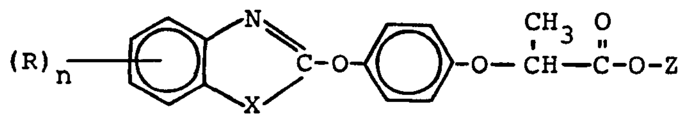
(I)

dans laquelle
R représente un halogène,
n est égal à 0, à 1 ou à 2,
X représente O ou S, et
Z représente un radical répondant à l'une des formules suivantes

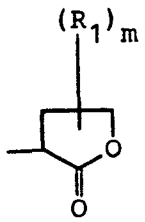

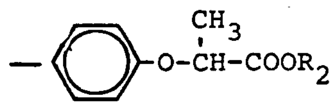

dans lesquelles
$R_1$ représente un radical alkyle en $C_1$-$C_4$,
m un nombre de 0 à 2 et
$R_2$ l'hydrogène, un alkyle en $C_1$-$C_4$ ou un équivalent de cation,
un radical morpholinométhyle ou un radical morpholinoéthyle.

2. Procédé pour préparer les composés de formule générale (I), procédé caractérisé en ce qu'on fait réagir

a) des composés répondant à la formule générale (II)

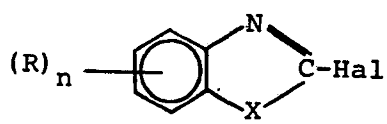
(II)

dans laquelle Hal représente un atome d'halogène, avec des composés répondant à la formule générale (III)

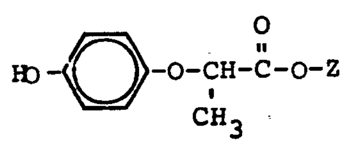
(III)

ou

b) des composés répondant à la formule générale (IV)

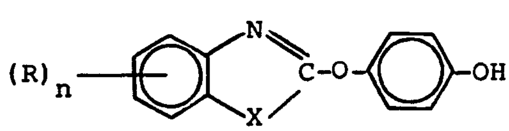
(IV)

avec des composés répondant à la formule générale (V)

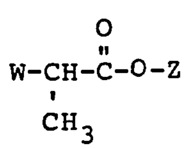
(V)

dans laquelle W représente un halogène, de préférence le chlore ou le brome, ou un radical tosyle, ou

c) des composés répondant à la formule générale (VI)

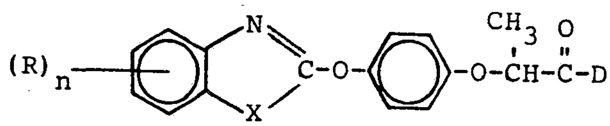 (VI)

avec des composés de formule B-Z (VII), l'un des symboles D et B représentant à chaque fois un halogène et l'autre un radical —OH.

3. Produits herbicides caractérisés en ce qu'ils contiennent des composés de formule générale (I).

4. Application de composés de formule générale (I) à la lutte contre des graminées nuisibles.

5. Procédé pour combattre des graminées adventices dans des plantes cultivées, procédé caractérisé en ce qu'on applique sur les graminées adventices ou sur leur espace vital une quantité efficace, du point de vue herbicide, d'un composé de formule générale (I).

**Revendications** (pour l'Etat contractant AT)

1. Procédé pour préparer des composés répondant à la formule générale (I)

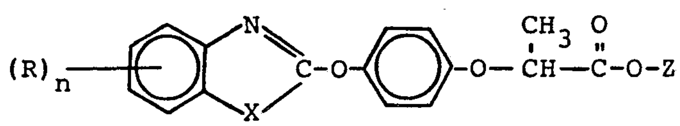 (I)

dans laquelle

R représente un halogène,

n est égal à 0, à 1 ou à 2,

X représente O ou S, et

Z représente un radical répondant à l'une des formule suivantes

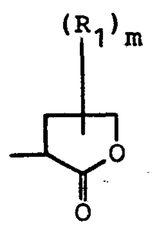 et 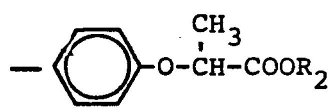

dans lesquelles

$R_1$ représente un radical alkyle en $C_1$-$C_4$,

m un nombre de 0 à 2 et

$R_2$ l'hydrogène, un alkyle en $C_1$-$C_4$ ou un équivalent de cation, un radical morpholinométhyle ou un radical morpholinoéthyle,

procédé caractérisé en ce qu'on fait réagir

a) des composés répondant à la formule générale (II)

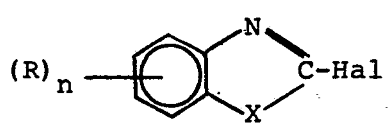 (II)

dans laquelle Hal représente un atome d'halogène, avec des composés répondant à la formule générale (III)

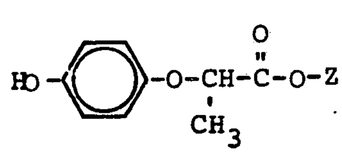 (III)

ou

b) des composés répondant à la formule générale (IV)

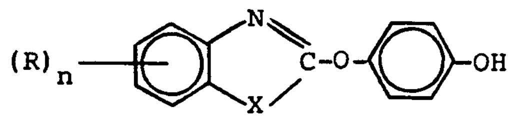

(IV)

avec des composés répondant à la formule générale (V)

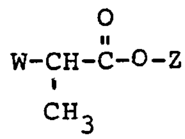

(V)

dans laquelle W représente un halogène, de préférence le chlore ou le brome, ou un radical tosyle, ou

c) des composés répondant à la formule générale (VI)

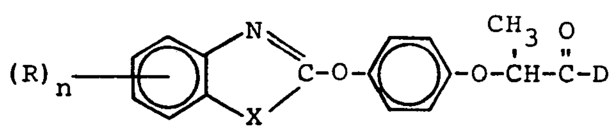

(VI)

avec des composés de formule B-Z (VII), l'un des symboles D et B représentant à chaque fois un halogène et l'autre un radical —OH.

2. Produits herbicides caractérisés en ce qu'ils contiennent des composés de formule générale (I).

3. Application de composés de formule générale (I) pour la lutte contre des graminées nuisibles.

4. Procédé pour combattre des graminées adventices dans des plantes cultivées, procédé caractérisé en ce qu'on applique sur les graminées adventices ou sur leur espace vital une quantité efficace, du point de vue herbicide, d'un composé de formule générale (I).